# EUROPEAN PATENT SPECIFICATION

(11) **EP 3 173 089 B1**
(45) Date of publication and mention of the grant of the patent: **25.10.2023**
(21) Application number: 15825197.5
(22) Date of filing: 23.07.2015
(51) Int. Cl.: A61K 31/23, A61K 31/7016, A61K 31/70, A61K 35/20, A61K 38/01, A61K 38/17, A61K 38/40, A61P 25/28, A23L 33/115, A23L 33/19, A23L 33/125, A23C 9/16

(54) **AGENT FOR IMPROVING BRAIN FUNCTION AND AGENT FOR PREVENTING COGNITIVE IMPAIRMENT**
MITTEL ZUR VERBESSERUNG DER GEHIRNFUNKTION UND MITTEL ZUR PRÄVENTION KOGNITIVER BEEINTRÄCHTIGUNG
AGENT D'AMÉLIORATION DE FONCTION CÉRÉBRALE ET AGENT POUR LA PRÉVENTION DU DÉFICIT COGNITIF

(30) Priority: 23.07.2014 JP 2014149990
(43) Date of publication of application: 31.05.2017
(73) Proprietor: The Food Science Institute Foundation, Odawara-shi, Kanagawa 250-0862 (JP); National Center of Neurology and Psychiatry, Kodaira-shi Tokyo 187-8551 (JP)
(72) Inventor: KUNUGI Hiroshi, Kodaira-shi Tokyo 187-8551 (JP); OTA Miho, Kodaira-shi Tokyo 187-8551 (JP); MATSUO Junko, Kodaira-shi Tokyo 187-8551 (JP); ICHIHARA Yoshitatsu, Odawara-shi Kanagawa 250-0862 (JP)
(74) Representative: Hoffmann Eitle
(86) International application number: PCT/JP2015/070976
(87) International publication number: WO 2016/013617

(56) References cited:
- WO-A1-2009/002145
- WO-A1-2014/027022
- WO-A2-01/19356
- WO-A2-2007/070701
- WO-A2-2007/115282
- JP-A- H06 287 138
- JP-A- 2009 524 587
- JP-A- 2009 532 496
- REGER M A ET AL: "Effect of .beta.-hydroxybutyrate on cognition in memory-impaired adults", NEUROBIOLOGY OF A, TARRYTOWN, NY, US, vol. 25, no. 3, 1 March 2004 (2004-03-01), pages 311-314, XP008122713, ISSN: 0197-4580, DOI: 10.1016/S0197-4580(03)00087-3
- YUANLONG PAN ET AL: "Dietary supplementation with medium-chain TAG has long-lasting cognition-enhancing effects in aged dogs", BRITISH JOURNAL OF NUTRITION, vol. 103, no. 12, 9 February 2010 (2010-02-09), pages 1746-1754, XP055449498, UK ISSN: 0007-1145, DOI: 10.1017/S0007114510000097
- CAROLYN VIALL ET AL: "A Double-Blind Clinical Trial Comparing the Gastrointestinal Side Effects of Two Enteral Feeding Formulas", JPEN - JOURNAL OF PARENTERAL AND ENTERAL NUTRITION, vol. 14, no. 3, 1 May 1990 (1990-05-01), pages 265-269, XP55449181, US ISSN: 0148-6071, DOI: 10.1177/0148607190014003265
- YEOU-MEI CHRISTIANA LIU ET AL.: 'Medium-chain triglyceride (MCT) ketogenic therapy' EPILEPSIA vol. 49, no. 8, 04 November 2008, pages 33 - 36, XP055389587 DOI: 10.1111/J.1528-1167.2008.01830.X
- OTA MIHO ET AL: "Effect of a ketogenic meal on cognitive function in elderly adults: potential for cognitive enhancement", PSYCHOPHARMACOLOGY, SPRINGER VERLAG, BERLIN, DE, vol. 233, no. 21, 27 August 2016 (2016-08-27), pages 3797-3802, XP036075510, ISSN: 0033-3158, DOI: 10.1007/S00213-016-4414-7 [retrieved on 2016-08-27]
- HAYASHI ANRI ET AL: "[Changes in serum levels of selenium, zinc and copper in patients on a ketogenic diet using Ketonformula].", NO TO HATTATSU = BRAIN AND DEVELOPMENT JUL 2013, vol. 45, no. 4, July 2013 (2013-07), pages 288-293, XP009518561, ISSN: 0029-0831
- NAKAZAWA MICHIHITO ET AL: "Effects of ketogenic diet on electroconvulsive threshold and brain contents of adenosine nucleotides", BRAIN AND DEVELOPMENT, vol. 5, no. 4, 1983, pages 375-380, XP028839248, ISSN: 0387-7604, DOI: 10.1016/S0387-7604(83)80042-4
- Anonymous: "KETOGENIC DIET WITH KETONFORMULA", , 20 June 2009 (2009-06-20), XP055762205, Retrieved from the Internet: URL:http://plaza.umin.ac.jp/~ketodiet/Keto nformulaKDforHP.pdf [retrieved on 2020-12-22]

## Description

### Technical Field

The present invention relates to a non-therapeutic use of an agent for the improvement of the brain function, an agent for use in preventing cognitive impairment, a non-therapeutic use of a composition for the improvement of the brain function, and a composition for use in preventing cognitive impairment.

### Background Art

It is known that brain function declines with age. The number of neurons in the brain generally decreases with age.

However, when cells are lost, new connections are sometimes established among the remaining neurons, or new neurons are sometimes created in some areas of the brain even in the aged stage. Also, because cells which are more than necessary for the most of the activities are present in the brain, the decrease can be partially compensated.

On the other hand, neurons tend to lose a part of the receptors for receiving messages due to aging, and blood flow to the brain tends to decrease. Such changes due to aging decrease brain function, and thus the aged sometimes take longer time to some extent for various kinds of response or for various operations although the aged can accurately response or conduct the operations when enough time is given. Moreover, it is said that some psychic functions such as vocabulary, short-term memory, capability of learning new things and capability of remembering words decline at the age of 70 or older.

With respect to such decline in brain function, reports suggest that intake of a composition which forms a ketone body in the body is effective also against memory impairment of the aged or Alzheimer's dementia. Ketone body is a generic term for acetone, acetoacetic acid and β-hydroxybutyric acid which are produced in the liver by the metabolism of fats and released into the blood.

In general, ketone bodies are formed to compensate the energy metabolism with the ketone bodies when there is a trouble in the metabolism of glucose, for example in the case of diabetes, high fat diet, fasting (starvation), an exercise, a traumatic injury or a major operation, and the ketone bodies are used as an energy source in the skeletal muscles, the heart, the kidneys and the like. The brain usually uses glucose, which can pass the blood-brain barrier, as the energy source. When glucose is depleted by fasting or the like, acetoacetic acid and β-hydroxybutyric acid also pass the blood-brain barrier like glucose and are used as energy in the TCA cycle in the mitochondria of the cells in the brain.

Of the compositions that form a ketone body in the body, medium-chain fatty acid triglycerides are known as effective components. For example, PTL 1 describes an agent for preventing or treating Alzheimer's disease containing a medium-chain fatty acid triglyceride derived from fatty acids having 8 to 10 carbon atoms as the main component.

The estimated proportion of the patients with dementia among the aged at the age of 65 or older is 15%, and the number in 2012 is estimated at about 4,620,000. Moreover, it is estimated that there are about 4,000,000 aged people who suffer from mild cognitive impairment, which may develop to dementia. This means that one fourth of the individuals at the age of 65 or older suffer from dementia or are candidates for dementia, and it is urgent that measures against dementia should be taken in the future.

Furthermore, WO 2007/115282 A2 is concerned with compositions for the treatment or prevention of loss of cognitive function caused by reduced neuronal metabolism in age-associated memory impairment (AAMI). In one embodiment, the composition includes medium chain triglycerides (MCT). The document also relates to oral dosage forms, in particular, a nutritional drink. According to an example, 20 g per day of Ketasyn^{™} (i.e. 50 wt.% of MCT and 50 wt.% of corn starch) mixed with 237 mL of the drink Ensure^{™} were administers to subjects suffering from AAMI. From day 31 on, each subject took 40 g of Ketasyn^{™}.

### Citation List

### Patent Literature

PTL 1: JP-A-6-287138

### Non Patent Literature

NPL 1: Henderson et al (2009) Nutr Metab. 2009;6;31

### Summary of Invention

### Technical Problem

In PTL 1, when the medium-chain fatty acid triglyceride is taken, it is necessary to consider diarrhea caused by the intake as disclosed in NPL 1.

Therefore, it is necessary to forcedly cause a medium-chain fatty acid triglyceride to be taken (administered or applied) to improve brain function, and the intake has a physical and/or psychological barrier.

Therefore, a problem to be solved by the invention is to provide a novel, safe and side-effect-free agent for improving brain function which can form a ketone body in the human body, and which does not cause diarrhea and/or nausea and is in a form that is easy to take. Also, a problem to be solved by the invention is to provide a novel, safe and side-effect-free agent for preventing or treating cognitive impairment which can form a ketone body in the human body, and which does not cause diarrhea and/or nausea and is in a form that is easy to take.

### Solution to Problem

The present inventors have conducted extensive investigation in view of the problems and made the following findings. That is, intake (administration or application) of an agent containing 12.5 to 20 parts by weight of a protein, which is at least one kind of protein selected from the group consisting of casein, a milk protein concentrate (MPC), a whey protein concentrate (WPC), a whey protein isolate (WPI), α-lactalbumin, β-lactoglobulin and lactoferrin, 30 to 50 parts by weight of a medium-chain fatty acid triglyceride as a lipid, and 7 to 12 parts by weight of a carbohydrate, which is at least one kind of carbohydrate selected from the group consisting of lactose, palatinose and trehalulose, per 100 parts by weight promotes the formation of a ketone body in the body and thus improves brain function and can prevent cognitive impairment. The problems have been thus solved. Specifically, the present invention relates to a non-therapeutic use of the agent for the improvement of the brain function in a healthy aged person, an agent for use in preventing cognitive impairment in a healthy aged person, a non-therapeutic use of a composition for the improvement of the brain function in a healthy aged person, and a composition for use in preventing cognitive impairment in a healthy aged person as defined in the appended claims.

Since the agents contain components which have been taken as food for a long time, such as a protein, a lipid and a carbohydrate, the agents are easily taken (administered or applied) continuously, are safe and carry a low risk of side effects.

Furthermore, when the agents are taken (administered or applied), a ketone body is formed in the body, and diarrhea and/or nausea are not caused. Thus, associated improvement of a symptom is expected. Examples of the cognitive impairment include mental retardation (dementia) as a cerebral disease, memory impairment, learning disability, consciousness disorder, cognitive disorder (agnosia), aphasia, apraxia, sleep disorder, headaches, movement disorder, sensory disorder, convulsive attack, anxiety neurosis as a mental disorder, hysteria, depression, hallucination, delusion and the like. It is believed that intake of the agents promotes the formation of a ketone body in the body and thus improves brain function and can prevent the cognitive impairment.

Also, because the agents are free of side effect and safe, a healthy aged person can also take the agents to improve brain function and to prevent cognitive impairment. The aged person in the invention means a person at the age of 60 or older unless otherwise specifically noted. The healthy aged person in the invention means an aged person who does not have the cognitive impairment.

That is, the invention is as follows.

According to a first aspect, the present invention relates to a non-therapeutic use of an agent for the improvement of the brain function in a healthy aged person, the agent comprising: 12.5 to 20 parts by weight of a protein; 30 to 50 parts by weight of a medium-chain fatty acid triglyceride as a lipid; and 7 to 12 parts by weight of a carbohydrate, per 100 parts by weight, wherein the protein is at least one kind of protein selected from the group consisting of casein, a milk protein concentrate (MPC), a whey protein concentrate (WPC), a whey protein isolate (WPI), α-lactalbumin,β-lactoglobulin and lactoferrin, and the carbohydrate is at least one kind of carbohydrate selected from the group consisting of lactose, palatinose and trehalulose.

A further aspect of the present invention relates to an agent for use in preventing cognitive impairment in a healthy aged person, wherein the agent is the above-defined agent.

A further aspect of the present invention relates to a non-therapeutic use of a composition for the improvement of the brain function in a healthy aged person, comprising the above-defined agent, wherein the composition is modified milk powder, liquid food, food for the sick, a supplement or enriched food.

A further aspect of the present invention relates to a composition for use in preventing cognitive impairment in a healthy aged person, comprising the above-defined agent, wherein the composition is modified milk powder, liquid food, food for the sick, a supplement or enriched food.

In particular, the agent does preferably not cause at least one of diarrhea and nausea.

### Advantageous Effects of Invention

Thus, a novel, safe and side-effect-free agent for the improvement of the brain function and a novel, safe and side-effect-free agent for use in preventing cognitive impairment which do not cause diarrhea and/or vomiting and which can form a ketone body in the human body in a form that is easy to take can be provided.

### Brief Description of Drawings

[Fig. 1] Fig. 1 shows the procedures of examination by the intake of the agent or a control.
[Fig. 2] Fig. 2 shows the relation between the elapsed time after the intake of the agent or the control and the blood acetoacetic acid concentration in Test Example 1.
[Fig. 3] Fig. 3 shows the relation between the elapsed time after the intake of the agent or the control and the blood β-hydroxybutyric acid concentration in Test Example 1.
[Fig. 4] Fig. 4 shows the relation between the improvement of the response time in the Trail Making Test-A after elapsed 1.5 hours and 3 hours after the intake of the agent and the blood β-hydroxybutyric acid concentration after elapsed 3 hours after the intake of the agent in Test Example 1.
[Fig. 5] Fig. 5 shows the relation between the elapsed time after the intake of the agent or the control and the blood acetoacetic acid concentration in Test Example 2.
[Fig. 6] Fig. 6 shows the relation between the elapsed time after the intake of the agent or the control and the blood β-hydroxybutyric acid concentration in Test Example 2.

### Description of Embodiments

The invention relates to a non-therapeutic use of an agent for the improvement of the brain function in a healthy aged person, the agent containing 12.5 to 20 parts by weight of a protein, 30 to 50 parts by weight of a medium-chain fatty acid triglyceride as a lipid and 7 to 12 parts by weight of a carbohydrate per 100 parts by weight or to a non-therapeutic use of a composition for the improvement of the brain function in a healthy aged person, the composition containing the agent for the improvement of the brain function. The protein is at least one kind of protein selected from the group consisting of casein, a milk protein concentrate (MPC), a whey protein concentrate (WPC), a whey protein isolate (WPI), α-lactalbumin, β-lactoglobulin and lactoferrin. The carbohydrate is at least one kind of carbohydrate selected from the group consisting of lactose, palatinose and trehalulose.

The invention also relates to an agent for use in preventing cognitive impairment in a healthy aged person, the agent containing 12.5 to 20 parts by weight of a protein, 30 to 50 parts by weight of a medium-chain fatty acid triglyceride as a lipid and 7 to 12 parts by weight of a carbohydrate per 100 parts by weight or to a composition for use in preventing cognitive impairment in a healthy aged person, the composition containing the agent for use in preventing cognitive impairment. The protein is at least one kind of protein selected from the group consisting of casein, a milk protein concentrate (MPC), a whey protein concentrate (WPC), a whey protein isolate (WPI), α-lactalbumin, β-lactoglobulin and lactoferrin. The carbohydrate is at least one kind of carbohydrate selected from the group consisting of lactose, palatinose and trehalulose.

In this description, the "agent for the improvement of the brain function" and the "agent for use in preventing cognitive impairment" are together referred to as "the agent", and the "composition for the improvement of the brain function" and the "composition for use in preventing cognitive impairment" are together referred to as "the composition", unless otherwise specifically noted.

### <Agent>

The agent contains 12.5 to 20 parts by weight of a protein, 30 to 50 parts by weight of a medium-chain fatty acid triglyceride as a lipid and 7 to 12 parts by weight of a carbohydrate per 100 parts by weight.

### <Protein>

The kind of protein contained in the agent is selected from casein, a milk protein concentrate (MPC), a whey protein concentrate (WPC), a whey protein isolate (WPI), α-lactalbumin, β-lactoglobulin and lactoferrin.

A kind or two or more kinds of the proteins can be used. When the protein is used, the medium-chain fatty acid triglyceride as a lipid can be taken easily.

Also, a commercial protein material can be used as long as the effects of the invention can be obtained. When the protein is used, the medium-chain fatty acid triglyceride as a lipid can be taken easily.

Moreover, the functional properties (physical properties such as solubility, viscosity, gelation, thermal stability and emulsion stability as well as physiological properties and the like) of the proteins may be modified according to the need through hydrolysis, modification of an amino acid residue or the like.

Furthermore, from the viewpoint of the inhibition of diarrhea, which is an effect of the invention, a fermented milk-derived protein which is expected to have an effect of regulating the gastric function and the like can also be used. As the material of the fermented milk protein, for example, a material obtained by fermenting milk with a lactic acid bacterium, a bifidobacterium and/or the like, such as yogurt or cheeses, can be used. Examples of a form of the fermented milk protein include fresh cheeses which are natural cheeses obtained by removing whey from fermented milk and which are not matured (quark, mascarpone, cream cheese and the like), and a kind or two or more kinds thereof can be used.

The amount of the protein added is 12.5 to 20 parts by weight per 100 parts by weight of the agent and can be appropriately adjusted within the range depending on the amounts of the other components (the medium-chain fatty acid triglyceride as a lipid and the carbohydrate), the pathological condition of the subject of the intake, the medical condition, the age, the body weight, the use or the like. The amount per 100 parts by weight of the composition is preferably 12.5 to 17.5 parts by weight. When the protein is a hydrolysate of whey protein, in particular, the amount can be preferably applied.

### <Medium-Chain Fatty Acid Triglyceride>

The kind of medium-chain fatty acid triglyceride (MCT) as a lipid contained in the agent is not particularly restricted. As the medium-chain fatty acid triglyceride, for example, triglycerides derived from fatty acids each having 5 to 12 carbon atoms, preferably 8 to 10 carbon atoms, and the like can be preferably used. When a triglyceride having 5 to 12 carbon atoms or the like is used, for example, a ketone body can be formed efficiently in the body of a human, and the effects of the invention can be further improved. A kind or two or more kinds of the medium-chain fatty acid triglycerides can be used.

As a saturated fatty acid having 8 to 10 carbon atoms, for example, caprylic acid, capric acid and the like can be used. A commercial edible fat or oil containing a medium-chain fatty acid triglyceride (for example, product name Nisshin MCT oil (manufactured by The Nisshin OilliO Group, Ltd.)) can be used as the medium-chain fatty acid triglyceride.

The amount of the medium-chain fatty acid triglyceride added is 30 to 50 parts by weight per 100 parts by weight of the agent and can be appropriately adjusted within the range depending on the amounts of the other components (the protein and the carbohydrate), the pathological condition of the subject of the intake, the medical condition, the age, the body weight, the use or the like.

The amount per 100 parts by weight is preferably 35 to 45 parts by weight.

### <Carbohydrate>

From the viewpoint of not increasing the blood sugar level after the intake, the carbohydrate is selected from lactose, palatinose, and trehalulose. When the carbohydrate is used, the medium-chain fatty acid triglyceride as a lipid can be taken easily. Also, to further improve the effects of the invention, a carbohydrate material which is expected to have an action of regulating the gastric function, such as dietary fiber, can be used. A kind or two or more kinds of the carbohydrates can be used.

The amount of the carbohydrate added is 7 to 12 parts by weight per 100 parts by weight of the agent and can be appropriately adjusted within the range depending on the amounts of the other components (the protein and the medium-chain fatty acid triglyceride as a lipid), the pathological condition of the subject of the intake, the medical condition, the age, the body weight, the use or the like. The amount per 100 parts by weight of the agent is preferably 8 to 10 parts by weight.

### <Other Components>

The agent can contain another fat or oil material as a lipid other than the above lipid, and the source and the kind thereof are not restricted. For example, a saturated fatty acid (palmitic acid, stearic acid or the like), a monovalent unsaturated fatty acid (oleic acid or the like), a polyvalent unsaturated fatty acid (linoleic acid, linolenic acid or the like), a phospholipid and the like can be used. Also, a long-chain fatty acid oil and fat (LCT) and the like can also be used.

As the phospholipid material, for example, one, two or more kinds of known phospholipid materials such as a milk phospholipid, soy lecithin and egg yolk lecithin can be used.

The phospholipid can be fractionated or purified from a source material such as milk, soybean, egg or the like. A commercial material containing a phospholipid can be used as long as the effects of the invention can be obtained.

The amount of the phospholipid added can be appropriately adjusted depending on the amounts of the other components (the protein, the medium-chain fatty acid triglyceride as a lipid and the carbohydrate), the pathological condition of the subject of the intake, the medical condition, the age, the body weight, the use or the like. For example, the amount of the milk phospholipid added is 0.01 to 1 part by weight per 100 parts by weight of the agent, preferably 0.05 to 0.8 parts by weight, more preferably 0.1 to 0.7 parts by weight, further preferably 0.15 to 0.6 parts by weight, further preferably 0.2 to 0.5 parts by weight, further preferably 0.25 to 0.45 parts by weight, particularly preferably 0.3 to 0.4 parts by weight.

With respect to the other fat or oil material, the amounts of a saturated fatty acid (palmitic acid, stearic acid or the like), a monovalent unsaturated fatty acid (oleic acid or the like) and a polyvalent unsaturated fatty acid (linoleic acid, linolenic acid or the like) added can be adjusted by comparing the standard of the intake of lipids set by the Ministry of Health, Labour and Welfare and the actual intake of lipids. Of the lipids, it is difficult to increase the amount of intake of a monovalent unsaturated fatty acid by the diet only in this country, and thus it is also preferable to increase the proportion of a monovalent unsaturated fatty acid in all the fatty acids.

Examples of the monovalent unsaturated fatty acid include oleic acid, palmitoleic acid, myristoleic acid and the like. Examples of a lipid source containing a high amount of oleic acid include high oleic sunflower oil containing a high amount of oleic acid, rapeseed oil, olive oil, high oleic safflower oil, soybean oil, corn oil, palm oil and the like. Also, a lipid source containing oleic acid is nutrient-modified fat or oil (manufactured by Nippon Oil & Fats Co., Ltd.). Moreover, sunflower oil, rapeseed oil, olive oil and a mixture with olive oil can also be used.

The amount of the other fat or oil material added can be appropriately adjusted depending on the amounts of the other components (the protein, the medium-chain fatty acid triglyceride as a lipid and the carbohydrate), the pathological condition of the subject of the intake, the medical condition, the age, the body weight, the use or the like. For example, the amount of high oleic sunflower oil containing a high amount of oleic acid added is 0 to 60 parts by weight per 100 parts by weight of the agent, preferably 5 to 55 parts by weight, more preferably 10 to 50 parts by weight, further preferably 15 to 45 parts by weight, further preferably 20 to 40 parts by weight, particularly preferably 25 to 35 parts by weight.

In addition, as materials of the agent, known food and food additive can be added for the purpose of making it easy to take the agent safely without a side effect. Moreover, for the purpose of improving the effects of the invention safely without a side effect, food and a food additive which improve the intestinal flora and which can inhibit diarrhea, such as known dietary fiber, can be added. Furthermore, for the purpose of improving the effects of the invention, food and a food additive which improve the flavor so that the agent becomes easy to take and which can inhibit vomiting can be added.

In addition to the protein, the lipid, the carbohydrate and the like, water and a known material that a human can take (that can be administered or applied to a human) can be added to the agent. For example, from the viewpoint of inhibiting a side effect, a food material, a food additive and the like which have been often used for food can be used. Moreover, the agent is in any form such as liquid, solid, powder or gel. The agent can be prepared by a known production method.

### <Method for Using Agent>

By applying an effective amount to a subject, the agent can exhibit the effect of improving brain function or the effect of preventing or treating cognitive impairment. Even by single intake (single administration), the agent is expected to exhibit the effect. In the case of a human of a body weight of 60 kg for example, the effective amount thereof as powder is 10 to 90 g a day, preferably 15 to 85 g, more preferably 20 to 80 g, further preferably 25 to 75 g, further preferably 30 to 70 g, further preferably 35 to 65 g, further preferably 40 to 60 g, particularly preferably 45 to 55 g. When the agent is taken (administered or applied), the powder which is dispersed or dissolved in an adequate amount of water in advance can be used. Moreover, the agent can be taken (administered or applied) by a known method such as an oral, transluminal or enteral method.

Because the agent does not cause a side effect such as diarrhea and/or nausea, the agent can be taken continuously, and a higher effect thereof is expected. In the case of a human of a body weight of 60 kg for example, the effective amount thereof as powder is the intake (administration or application) of 10 g or more a day, 15 g or more a day, 20 g or more a day, 25 g or more a day, 30 g or more a day, 35 g or more a day, 40 g or more a day, 45 g or more a day or 50 g or more a day. Also, in the case of a human of a body weight of 60 kg for example, the intake (administration or application) is for one day or longer, two days or longer, three days or longer, four days or longer, five days or longer, six days or longer, one week or longer, two weeks or longer, three weeks or longer, four weeks or longer, six weeks or longer, eight weeks or longer, 12 weeks or longer or 24 weeks or longer. Moreover, for example, continuous intake is once a week, twice a week, three times a week, four times a week, five times a week, six times a week or seven times a week. In particular, application of 10 g or more a day is preferable.

The agent can be taken by a healthy aged person.

Examples of the cognitive impairment include mental retardation (dementia) as a cerebral disease, memory impairment, learning disability, consciousness disorder, cognitive disorder (agnosia), aphasia, apraxia, sleep disorder, headaches, movement disorder, sensory disorder, convulsive attack, anxiety neurosis as a mental disorder, hysteria, depression, hallucination, delusion and the like. The agent can be used for a healthy aged person and can improve brain function.

Because the agent is free of side effect and safe, the agent can be used also as an agent for use in preventing cognitive impairment for a healthy aged person.

### <Composition >

The agent can be applied alone but can also be used as a composition containing the agent (the composition). That is, the agent can be used for the manufacture of the composition. The composition can be used for the improvement of brain function or for the prevention of cognitive impairment in a healthy aged person.

When the composition is produced, the amount of the agent added to the composition can be optionally determined depending on the purpose, the use, the form, the dosage form, the symptom, the body weight and the like. Although the invention is not limited thereto, as the amount thereof relative to the total amount, the agent can be added in an amount of 5 to 95% (w/w) and can be added further preferably in an amount of 10 to 50% (w/w). This is because the intake (administration or application) becomes easy in the range.

Specifically, the composition can be used in the form of food or drink (a food or drink composition). For example, the composition is expected to exhibit the effect of improving brain function or the effect of preventing or treating cognitive impairment by directly taking the composition as food for special dietary uses such as foods for specified health uses or nutritional food. The food for special dietary uses and the nutritional food are selected from modified milk powder, liquid food, food for the sick, a supplement, and enriched food.

When the composition is prepared as a food or drink composition without a side effect, the agent may be added to various foods and drinks, namely modified milk powder, liquid food, food for the sick, a supplement or enriched food and then taken. The agent can be used according to a normal method for general food or drink compositions, for example by directly using the agent or mixing with other food or food components.

The state thereof may be any generally used state of food or drink such as solid (powder, granules and the like), paste, liquid or suspension. In such a form, the composition can be taken without a psychological problem.

When the composition is provided as a food composition, the production method thereof may be a method known to one skilled in the art. One skilled in the art can produce a desired food by appropriately combining a step of mixing the agent with other components, a forming step, a sterilization step, a fermentation step, a calcination step, a drying step, a cooling step, a granulation step, an enclosing step and the like.

The composition can be applied also to food with health claims or food for the sick. The system for food with health claims has been established not only for general foods but also for foods in the form of tablets, capsules and the like, in view of the trends inside and outside of Japan and also considering the consistency with the existing system for foods for specified health uses. The system for food with health claims includes two types, namely foods for specified health uses (individual approval system) and foods with nutrient function claims (standard regulation system). It is expected that the effect of improving brain function or the effect of preventing or treating cognitive impairment is exhibited when the composition is directly taken as food for special dietary uses such as foods for specified health uses or food with nutrient function claims.

The subject of the intake (administration or application) of the composition is similar to that of the agent. The composition can be taken by a healthy aged person.

Examples of the cognitive impairment include mental retardation (dementia) as a cerebral disease, memory impairment, learning disability, consciousness disorder, cognitive disorder (agnosia), aphasia, apraxia, sleep disorder, headaches, movement disorder, sensory disorder, convulsive attack, anxiety neurosis as a mental disorder, hysteria, depression, hallucination, delusion and the like. The composition can be used for a healthy aged person and can improve brain function.

Because the composition is free of side effect and safe, the composition can be used also for the prevention of cognitive impairment for a healthy aged person.

### Examples

The invention is explained in further detail below referring to Examples, but the invention is not limited by the Examples.

### (Test Example 1) Experiment 1 of Administration of Agent to Healthy Aged Individuals [Production of Agent]

Powder containing a medium-chain fatty acid triglyceride (MCT) was obtained by blending a composition containing 40% by weight of the medium-chain fatty acid triglyceride, 30.1% by weight of a long-chain fatty acid oil and fat (LCT), 0.4% by weight of soy lecithin, 8% by weight of lactose, 0.2% by weight of citric acid, 14% by weight of casein, 4% by weight of a whey protein concentrate 0.2% by weight of vitamins and 3.1% by weight of minerals with water for dissolution, emulsifying the solution by homogenization and spray drying the solution. A solution obtained by dissolving 50 g of the powder in 79 g of water (total 129 g) was used as "the agent" below.

As a control, 825 g of vegetable fat cream for whipping ("Whip" manufactured by Megmilk Snow Brand Co., Ltd.), 400 g of cow's milk and 62.5 g of protein powder ("Meiji Mei Protein Zn" manufactured by Meiji Co., Ltd.) were mixed, blended and emulsified by homogenization. The obtained material was used as "the control" below.

### [Selection of Subjects]

Of the individuals at the age of 60 or older who were recruited using a website, leaflets and the like, two males and five females who gave written consent (the average age was 65.9±3.1 years old) were selected as subjects. The seven subjects did not have any medical diseases such as impaired liver/kidney function, hyperlipidemia and diabetes and did not have any organic brain disease. The seven subjects consulted with a doctor psychiatrically as to whether the subjects had cognitive impairment, and it was confirmed that the subjects did not suffer from any mental diseases.

### [Intake of Agent]

A crossover study was conducted using "the agent" and "the control" using the seven subjects, and the increases in the blood ketone body concentrations and the effect of improving cognitive function were examined.

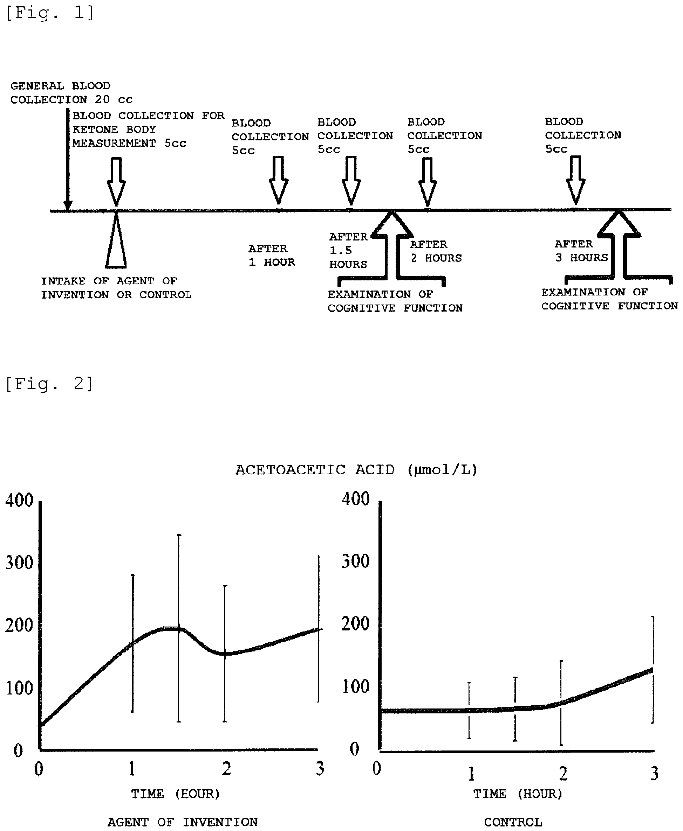

Specifically, first, the subjects fasted after 22 o'clock on the day before the examination, and the examination was started at 9 o'clock on the day of the examination. Blood samples were collected from the vein, and the liver function, the kidney function, the fasting blood sugar level, the cholesterol value and the like were evaluated. At 10 o'clock on the same day, 129 g of the agent produced above (the solution obtained by dissolving 50 g of the powder (containing 20 g of the medium-chain fatty acid) in 79 g of water) was taken once, and 129 g of the control (corresponding to one in which the medium-chain fatty acid component was replaced with another fatty acid) was taken once on another day. The order of "the agent" and "the control" was determined by a simple randomized method. Blood samples were collected from the vein 1, 1.5, 2 and 3 hours after the start of the intake, and the blood ketone body concentrations (acetoacetic acid and β-hydroxybutyric acid in the blood) were measured. The cognitive function was evaluated 1.5 hours and 3 hours after the intake by the Trail Making Test (document: Lezak MD, Howieson DB, Loring DW. Neuropsychological Assessment. NY Oxford University Press 2004.), which examines maintenance of attention, selection and visual search/visual-motor coordination (Fig. 1). The subjects were asked about any adverse events such as diarrhea and nausea during the examination.

### [Evaluation]

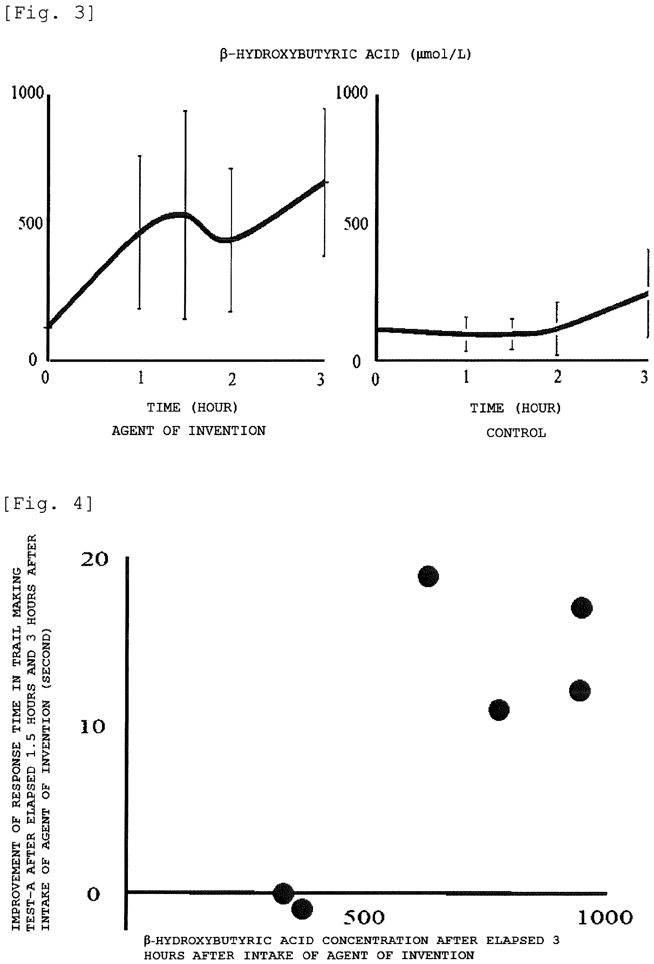

As a result of the measurement of the blood ketone body concentrations, it was confirmed that the acetoacetic acid concentration (Fig. 2) and the β-hydroxybutyric acid concentration (Fig. 3) in the blood increased with time after the intake of "the agent" as compared to the concentrations after the intake of "the control".

When the scores of the Trail Making Test-A were examined, in the case of the intake of "the control", the average response times were 29.0±7.5 seconds 1.5 hours after the intake of the control and 29.1±13.6 seconds 3 hours after the intake of the control, and an effect of the intake of the control (a decrease in the average response time) was not observed.

On the other hand, in the case of the intake of "the agent", the average response times were 34.6±7.8 seconds 1.5 hours after the intake of the agent and 25.1±4.7 seconds 3 hours after the intake of the agent. An effect of the intake of the agent (a decrease in the average response time) was observed, and the scores of the Trail Making Test-A improved as the blood ketone body concentrations increased (Fig. 4).

The above results suggest that the agent has the action of improving cognitive function in healthy aged individuals. In this regard, no serious side effect was observed in the series of examination. Transient nausea was observed after the intake of the agent in only one case of the seven cases, but the symptom disappeared quickly. In any of the seven cases, symptom of diarrhea was not observed after the intake of the agent.

### (Test Example 2) Experiment 2 of Administration of Agent to Healthy Aged Individuals [Production of Agent]

"The agent" and "the control" were produced in similar manners to those of Test Example 1.

### [Selection of Subjects]

Of the individuals at the age of 60 or older who were recruited using a website, leaflets and the like, six males and 14 females who gave written consent (the average age was 66.3±2.9 years old) were selected as subjects. The 20 subjects did not have any medical diseases such as impaired liver/kidney function, hyperlipidemia and diabetes and did not have any organic brain disease. The 20 subjects consulted with a doctor psychiatrically as to whether the subjects had cognitive impairment, and it was confirmed that the subjects did not suffer from any mental diseases.

### [Intake of Agent]

In a similar manner to that of Test Example 1, a crossover study was conducted using "the agent" and "the control" using the subjects, and the increases in the blood ketone body concentrations and the effect of improving cognitive function were examined. One and a half hours and three hours after the intake of "the agent" or "the control", the cognitive function was evaluated by the Trail Making Test (document: Lezak MD, Howieson DB, Loring DW. Neuropsychological Assessment. NY Oxford University Press 2004.), which examines maintenance of attention, selection and visual search/visual-motor coordination, and the concentration and the memory retention were evaluated by the digit span assessment and the visual memory span assessment of the Japanese-version Wechsler Memory Scale (document: Wechsler D. Wechsler memory scale-revised. 1987; San Antonio; Psychological Corporation.) (Fig. 1). The subjects were asked about any adverse events such as diarrhea and nausea during the examination.

### [Evaluation]

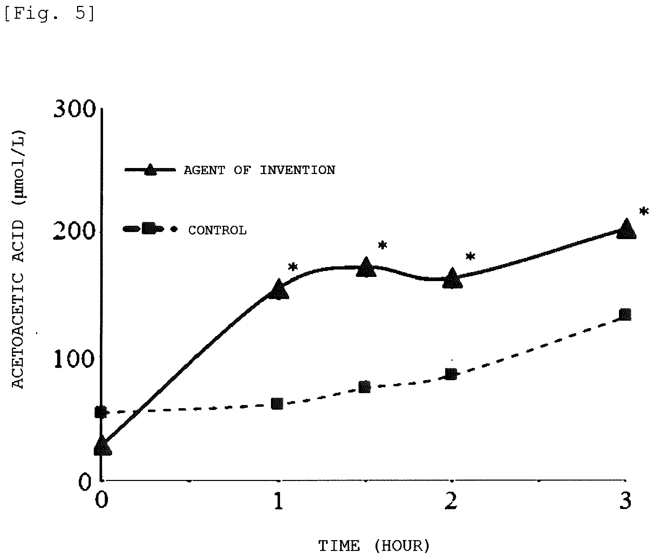

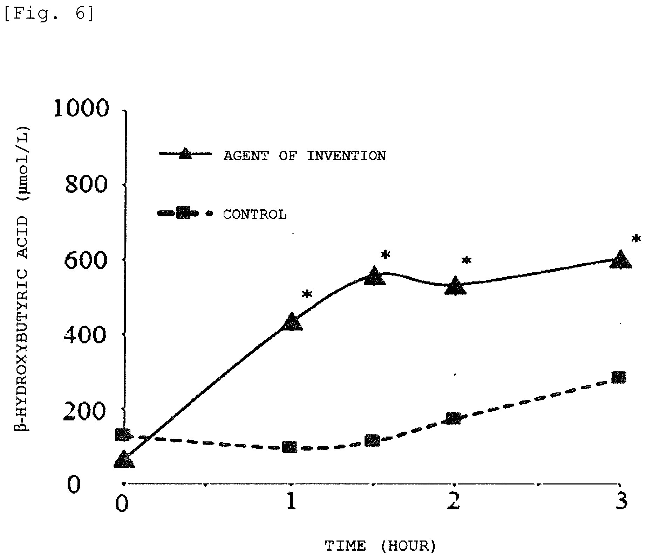

As a result of the measurement of the blood ketone body concentrations, it was confirmed that the acetoacetic acid concentration (Fig. 5) and the β-hydroxybutyric acid concentration (Fig. 6) in the blood increased with time after the intake of "the agent" as compared to the concentrations when "the control" was taken.

When the scores of the Trail Making Test-B were examined, in the case of the intake of "the control", the average response times were 77.8±23.7 seconds 1.5 hours after the intake of the control and 79.0±28.9 seconds 3 hours after the intake of the control, and an effect of the intake of the control (a decrease in the average response time) was not observed.

On the other hand, in the case of the intake of "the agent", the average response times were 75.0±18.9 seconds 1.5 hours after the intake of the agent and 70.7±26.6 seconds 3 hours after the intake of the agent. An effect of the intake of the agent (a decrease in the average response time) was observed, and the scores of the Trail Making Test-B improved as the blood ketone body concentrations increased.

Moreover, when the scores of the digit span assessment of the Japanese-version Wechsler Memory Scale were examined, the average forward and backward digit span in the case of the intake of "the control" was 11.6±3.5 digits 1.5 hours after the intake of the control, but the average forward and backward digit span in the case of the intake of "the agent" was 12.5±3.2 digits 1.5 hours after the intake of the agent. Thus, significant improvement was observed.

As the results of Test Example 1, the above results also suggest that the agent has the action of improving cognitive function in healthy aged individuals. In this regard, no serious side effect was observed in the series of examination. In any of the 20 cases, symptom of diarrhea was not observed after the intake of the agent.

### (Test Example 3) Experiment of Administration of Agent to Patients with Dementia (Reference Example, not according to the claimed invention).

### [Production of Agent]

"The agent" and "the control" were produced in similar manners to those of Test Example 1.

### [Selection of Subjects]

In this test, two patients with dementia (Subject 1, a 70-year-old male, and Subject 2, a 67-year old male) were selected as the subjects.

### [Intake of Agent by Subjects]

In a similar manner to that of Test Example 1, a crossover study was conducted using "the agent" and "the control" using the subjects, and the blood ketone body concentrations were measured. The specific method for taking "the agent" or "the control" was similar to that of Test Example 1. Blood samples were collected from the vein before the intake of "the agent" or "the control" and two hours after the intake, and the blood ketone body concentrations (acetoacetic acid, β-hydroxybutyric acid and the total of the ketone bodies in the blood) were measured.

### [Evaluation]

The measurement results of Subject 1 are shown in Table 1, and the measurement results of Subject 2 are shown in Table 2.

In both subjects, it was confirmed that the acetoacetic acid concentration, the β-hydroxybutyric acid concentration and the total ketone body concentration in the blood increased significantly after the intake of "the agent" as compared to the concentrations after the intake of "the control". The results suggested that "the agent" is effective for preventing or treating cognitive impairment such as dementia. In this regard, no serious side effect was observed in the series of examination. In either of the two cases, symptom of diarrhea was not observed after the intake of the agent.

### [Table 1]

**Table 1**

| | Acetoacetic acid (µmol/L) | β-hydroxybutyric acid (µmol/L) | Total of ketone bodies (µmol/L) |
|---|---|---|---|
| Before the intake of the agent | 42 | 93 | 135 |
| Two hours after the intake of the agent | 86 | 330 | 416 |
| Before the intake of the control | 28 | 57 | 85 |
| Two hours after the intake of the control | 14 | 23 | 37 |

### [Table 2]

**Table 2**

| | Acetoacetic acid (µmοl/L) | β-hydroxybutyric acid (µmol/L) | Total of ketone bodies (µmol/L) |
|---|---|---|---|
| Before the intake of the agent | 35 | 43 | 78 |
| Two hours after the intake of the agent | 87 | 271 | 358 |
| Before the intake of the control | 3 | 51 | 54 |
| Two hours after the intake of the control | 8 | 92 | 100 |

### Industrial Applicability

When the agent is taken (administered or applied), a ketone body is formed in the body, and diarrhea and/or nausea are not caused. Thus, associated improvement of a symptom is expected. An example is the improvement of cognitive function of an aged person, such as mental retardation (dementia) as a cerebral disease, memory impairment, learning disability, consciousness disorder, cognitive disorder (agnosia), aphasia, apraxia, sleep disorder, headaches, movement disorder, sensory disorder, convulsive attack, anxiety neurosis as a mental disorder, hysteria, depression, hallucination or delusion. Moreover, because the agent is free of side effect and safe, the agent can be taken also as an agent for preventing dementia for a healthy aged person for example.

## Claims

1. Non-therapeutic use of an agent for the improvement of the brain function in a healthy aged person, the agent comprising: 12.5 to 20 parts by weight of a protein; 30 to 50 parts by weight of a medium-chain fatty acid triglyceride as a lipid; and 7 to 12 parts by weight of a carbohydrate, per 100 parts by weight,
wherein the protein is at least one kind of protein selected from the group consisting of casein, a milk protein concentrate (MPC), a whey protein concentrate (WPC), a whey protein isolate (WPI), α-lactalbumin, β-lactoglobulin and lactoferrin, and
the carbohydrate is at least one kind of carbohydrate selected from the group consisting of lactose, palatinose and trehalulose.

2. An agent for use in preventing cognitive impairment in a healthy aged person, the agent comprising: 12.5 to 20 parts by weight of a protein; 30 to 50 parts by weight of a medium-chain fatty acid triglyceride as a lipid; and 7 to 12 parts by weight of a carbohydrate, per 100 parts by weight,
wherein the protein is at least one kind of protein selected from the group consisting of casein, a milk protein concentrate (MPC), a whey protein concentrate (WPC), a whey protein isolate (WPI), α-lactalbumin, β-lactoglobulin and lactoferrin, and
wherein the carbohydrate is at least one kind of carbohydrate selected from the group consisting of lactose, palatinose and trehalulose.

3. Non-therapeutic use of a composition for the improvement of the brain function in a healthy aged person, comprising the agent for the improvement of the brain function as defined in claim 1, wherein the composition is modified milk powder, liquid food, food for the sick, a supplement or enriched food.

4. A composition for use in preventing cognitive impairment in a healthy aged person, comprising the agent for use in preventing cognitive impairment as defined in claim 2, wherein the composition is modified milk powder, liquid food, food for the sick, a supplement or enriched food.

## Patentansprüche

1. Nicht-therapeutische Verwendung eines Mittels zur Verbesserung der Gehirnfunktion bei einer gesunden älteren Person, wobei das Mittel umfasst: 12,5 bis 20 Gewichtsteile eines Proteins; 30 bis 50 Gewichtsteile eines Fettsäuretriglycerids mittlerer Kettenlänge als Lipid; und 7 bis 12 Gewichtsteile eines Kohlenhydrats, pro 100 Gewichtsteile,
wobei das Protein mindestens eine Art von Protein ist, das aus der Gruppe ausgewählt ist, die aus Casein, einem Milchproteinkonzentrat (MPC), einem Molkenproteinkonzentrat (WPC), einem Molkenproteinisolat (WPI), α-Lactalbumin, β-Lactoglobulin und Lactoferrin besteht, und
wobei das Kohlenhydrat mindestens eine Art von Kohlenhydrat ist, das aus der Gruppe ausgewählt ist, die aus Lactose, Palatinose und Trehalulose besteht.

2. Mittel zur Verwendung bei der Vorbeugung von kognitiver Beeinträchtigung bei einer gesunden älteren Person, wobei das Mittel umfasst: 12,5 bis 20 Gewichtsteile eines Proteins; 30 bis 50 Gewichtsteile eines Fettsäuretriglycerids mittlerer Kettenlänge als Lipid; und 7 bis 12 Gewichtsteile eines Kohlenhydrats, pro 100 Gewichtsteile,
wobei das Protein mindestens eine Art von Protein ist, das aus der Gruppe ausgewählt ist, die aus Casein, einem Milchproteinkonzentrat (MPC), einem Molkenproteinkonzentrat (WPC), einem Molkenproteinisolat (WPI), α-Lactalbumin, β-Lactoglobulin und Lactoferrin besteht, und
wobei das Kohlenhydrat mindestens eine Art von Kohlenhydrat ist, das aus der Gruppe ausgewählt ist, die aus Lactose, Palatinose und Trehalulose besteht.

3. Nicht-therapeutische Verwendung einer Zusammensetzung zur Verbesserung der Gehirnfunktion bei einer gesunden älteren Person, umfassend das Mittel zur Verbesserung der Gehirnfunktion, wie in Anspruch 1 definiert, wobei die Zusammensetzung ein modifiziertes Milchpulver, ein flüssiges Nahrungsmittel, ein Nahrungsmittel für Kranke, ein Ergänzungsmittel oder ein angereichertes Nahrungsmittel ist.

4. Zusammensetzung zur Verwendung bei der Vorbeugung von kognitiver Beeinträchtigung bei einer gesunden älteren Person, umfassend das Mittel zur Verwendung bei der Vorbeugung von kognitiver Beeinträchtigung, wie in Anspruch 2 definiert, wobei die Zusammensetzung ein modifiziertes Milchpulver, ein flüssiges Nahrungsmittel, ein Nahrungsmittel für Kranke, ein Ergänzungsmittel oder ein angereichertes Nahrungsmittel ist.

## Revendications

1. Utilisation non thérapeutique d'un agent d'amélioration de la fonction cérébrale chez une personne âgée en bonne santé, l'agent comprenant : 12,5 à 20 parties en poids d'une protéine ; 30 à 50 parties en poids d'un triglycéride d'acide gras à chaîne moyenne en tant que lipide ; et 7 à 12 parties en poids d'un glucide, pour 100 parties en poids,
dans laquelle la protéine est au moins un type de protéine sélectionnée dans le groupe consistant en la caséine, un concentré de protéines de lait (MPC), un concentré de protéines de lactosérum (WPC), un isolat de protéines de lactosérum (WPI), l'a-lactalbumine, la β-lactoglobuline et la lactoferrine, et
le glucide est au moins un type de glucide sélectionné dans le groupe consistant en le lactose, le palatinose et le tréhalulose.

2. Agent destiné à être utilisé dans la prévention des troubles cognitifs chez une personne âgée en bonne santé, l'agent comprenant : 12,5 à 20 parties en poids d'une protéine ; 30 à 50 parties en poids d'un triglycéride d'acide gras à chaîne moyenne en tant que lipide ; et 7 à 12 parties en poids d'un glucide, pour 100 parties en poids,
dans lequel la protéine est au moins un type de protéine sélectionnée dans le groupe consistant en la caséine, un concentré de protéines de lait (MPC), un concentré de protéines de lactosérum (WPC), un isolat de protéines de lactosérum (WPI), l'α-lactalbumine, la β-lactoglobuline et la lactoferrine, et
dans lequel le glucide est au moins un type de glucide sélectionné dans le groupe consistant en le lactose, le palatinose et le tréhalulose.

3. Utilisation non thérapeutique d'une composition pour l'amélioration de la fonction cérébrale chez une personne âgée en bonne santé, comprenant l'agent d'amélioration de la fonction cérébrale tel que défini dans la revendication 1, dans laquelle la composition est un lait en poudre modifié, un aliment liquide, un aliment pour malades, un complément ou un aliment enrichi.

4. Composition destinée à être utilisée dans la prévention des troubles cognitifs chez une personne âgée en bonne santé, comprenant l'agent destiné à être utilisé dans la prévention des troubles cognitifs tel que défini dans la revendication 2, dans laquelle la composition est un lait en poudre modifié, un aliment liquide, un aliment pour malades, un complément ou un aliment enrichi.
